(19) Europäisches Patentamt
European Patent Office
Office européen des brevets

(11) Veröffentlichungsnummer : **0 437 900 B1**

(12) # EUROPÄISCHE PATENTSCHRIFT

(45) Veröffentlichungstag der Patentschrift : **02.03.94 Patentblatt 94/09**

(51) Int. Cl.$^5$ : **A01N 43/36**

(21) Anmeldenummer : **90250346.5**

(22) Anmeldetag : **28.12.90**

(54) **Desinfektionsmittellösung zur verbesserten Keimabtötung.**

(30) Priorität : **28.12.89 DD 336603**

(43) Veröffentlichungstag der Anmeldung : **24.07.91 Patentblatt 91/30**

(45) Bekanntmachung des Hinweises auf die Patenterteilung : **02.03.94 Patentblatt 94/09**

(84) Benannte Vertragsstaaten : **AT CH DE FR IT LI NL**

(56) Entgegenhaltungen :
**EP-A- 0 202 349**
**DD-A- 278 062**

(73) Patentinhaber : **Ohme, Roland, Dr. Dipl.-Chem.
Grünau, Waldstrasse 6
D-12527 Berlin (DE)**
Patentinhaber : **Gründemann, Egon, Dr. Dipl.-Chem.
Waldstrasse 4
D-12527 Berlin (DE)**
Patentinhaber : **Ballschuh, Detlef, Dr. Dipl.-Chem.
Graudenzer Strasse 17
D-10243 Berlin (DE)**
Patentinhaber : **Seibt, Horst, Dr. Dipl.-Chem.
Eugen-Schönhaar-Strasse 15
D-10407 Berlin (DE)**
Patentinhaber : **Kramer, Axel, Prof.Dr.med.habil.
Georg-Engel-Strasse 20
D-17489 Greifswald (DE)**
Patentinhaber : **Fischer, Ingeborg, Dipl.-Ing.
Schillingstrasse 24
D-10179 Berlin (DE)**
Patentinhaber : **Nordheim, Willy, Dr.habil. Dipl.-Ing.
Loeferweg 22
D-12681 Berlin (DE)**
Patentinhaber : **Bräuninger, Siegfried, Dr. Dipl.-Biol.
Berliner Strasse 65
D-13189 Berlin (DE)**
Patentinhaber : **Krüger, Erika
Heinestrasse 8
D-15745 Wildau (DE)**
Patentinhaber : **Weuffen, Wolfgang,
Prof.Dr.sc.med.Dr.rer.nat.
Ringstrasse 39
D-17498 Guest (DE)**
Patentinhaber : **Jülich, Wolf-Dieter,
Dr.sc.Dipl.-Chem.
Wollweberstrasse 9
D-17489 Greifswald (DE)**

(72) Erfinder : **Ohme, Roland, Dr. Dipl.-Chem.
Grünau, Waldstrasse 6
D-12527 Berlin (DE)**
Erfinder : **Gründemann, Egon, Dr. Dipl.-Chem.
Waldstrasse 4
D-12527 Berlin (DE)**
Erfinder : **Ballschuh, Detlef, Dr. Dipl.-Chem.
Graudenzer Strasse 17
D-10243 Berlin (DE)**
Erfinder : **Seibt, Horst, Dr. Dipl.-Chem.
Eugen-Schönhaar-Strasse 15
D-10407 Berlin (DE)**
Erfinder : **Kramer, Axel, Prof.Dr.med.habil.
Georg-Engel-Strasse 20
D-17489 Greifswald (DE)**
Erfinder : **Fischer, Ingeborg, Dipl.-Ing.
Schillingstrasse 24
D-10179 Berlin (DE)**
Erfinder : **Nordheim, Willy, Dr.habil. Dipl.-Ing.
Loeferweg 22
D-12681 Berlin (DE)**
Erfinder : **Bräuninger, Siegfried, Dr. Dipl.-Biol.
Berliner Strasse 65
D-13189 Berlin (DE)**
Erfinder : **Krüger, Erika
Heinestrasse 8
D-15745 Wildau (DE)**
Erfinder : **Weuffen, Wolfgang,
Prof.Dr.sc.med.Dr.rer.nat.
Ringstrasse 39
D-17498 Guest (DE)**
Erfinder : **Jülich, Wolf-Dieter, Dr.sc.Dipl.-Chem.
Wollweberstrasse 9
D-17489 Greifswald (DE)**

(74) Vertreter : **Hübner, Annegret et al
Hübner - Neumann - Radwer Frankfurter Allee 286
O-10317 Berlin (DE)**

EP 0 437 900 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Anwendungsgebiet der Erfindung

Die Anwendung bezieht sich auf eine Keimabtötung/Inaktivierung von Keimen mit breiter Wirkung im human- und veterinärmedizinischen Bereich sowie in der Lebensmittelindustrie.

Die Anwendung ist für Einrichtungen möglich, in denen Maßnahmen für eine multivalente Keimminderung, eine keimmindernde Reinigung bzw. Desinfektion oder Sterilisation notwendig sind.

Charakteristik des bekannten Standes der Technik

Multivalent wirkende Mittel zur Keimabtötung unter gleichzeitiger Beachtung der Anwenderfreundlichkeit, einer hohen Effektivität sowie von toxikologischen und ökologischen Kriterien und Berücksichtigung des Kapillarproblems und nicht zuletzt einer günstigen Ökonomie existieren bisher noch nicht. Aufgrund der Verschiedenartigkeit der Keime, der Anwendungsgebiete, Produkte und Prozesse oder der Verträglichkeit und Umweltbelastung durch Desinfektionsmittel ist das Gebiet sehr unübersichtlich und verbesserungsbedürftig. Eine Giederung nach chemischen Stoffklassen mit Bezug auf die entsprechenden Anwendungsgebiete ist auch schwer möglich. Andererseits werden wegen der Unterschiedlichkeit der Keime die eingesetzten Desinfektionsmittel auch variabel gehalten, und die chemischen Wirkstoffe kommen oft untereinander kombiniert oder zusammen mit physikalischen Mitteln (z. B. Temperaturerhöhung) zum Einsatz.

Wegen der Vielzahl der Anwendungskriterien sind daher für die einzelnen Desinfektionsverfahren und besonders Desinfektionsaufgaben (Hände-, Haut-, Instrumentendesinfektion usw.) die einzelnen Desinfektionsmittel angepaßt. Die Forderungen nach schneller und sicherer Desinfektion sind schwer in Einklang zu bringen mit der Forderung nach Verträglichkeit, der Forderung nicht korrosiv zu wirken und anderen ökologischen und ökonomischen Gesichtspunkten wie Haltbarkeit, Lagerfähigkeit, Geruchsbelästigung, Hautirritation und Handhabbarkeit. Nach dem Stand der Technik werden hohe Wirksamkeit und Sicherheit von Nachteilen in der Handhabung begleitet. Bei der Suche nach neuen, sicheren Desinfektionsmitteln sind antivirale Mittel bisher nicht umfassend genug bearbeitet worden, wodurch die oben genannten Nachteile für die Virusdesinfektion in besonderem Maße bestehen und die Palette der einsetzbaren antiviralen Desinfektionsmittel noch klein ist. Zur chemischen Keimabtötung wurden bisher folgende Mittel angewandt (HORN, PRIVORA, WEUFFEN, "Handbuch der Desinfektion und Sterilisation" Band 1 bis 5, Verlag Volk und Gesundheit, Berlin 1972-1984):

- Aldehyde bzw. Gemische von Aldehyden;
- Alkohole, besonders Ethanol und die Propanole;
- Halogenverbindungen, besonders Jodverbindungen und Hypochlorite;
- Lactone;
- Metallverbindungen (Quecksilberchlorid, Kupfersulfat);
- Oxydantien (Peressigsäure, Ozon), Epoxide;
- Phenole, Xylenole;
- Quartäre Ammoniumverbindungen;
- spezielle Tenside, z. B. Carbobetaine,

wobei sich neue Wirkstofftypen für Desinfektionsmittel bisher nicht durchgesetzt haben.

Von den genannten Verbindungen haben z. B. Peressigsäure und Aldehyde einen sehr breiten Wirkungsbereich. Diese Substanzen sind aber in der Regel toxisch, lagerinstabil und/oder weisen hohe Korrosivität auf. Dazu kommt eine rasche Inaktivierung der Peressigsäure durch Blut, was zu einer erheblichen Wirkungseinschränkung führt. Formaldehyd führt aufgrund seiner sensibilisierenden Wirkung zu Berufserkrankungen. Glutardialdehyd zeigt beträchtliche metallkorrodierende Wirkung und ist wegen geringer Haltbarkeit zur Allgemeindesinfektion wenig geeignet; auch werden Kontaktdermatitiden verursacht (Lexikon der Hilfsstoffe für Pharmazie und Kosmetik, Editio Cantor Aulendorf, 2. Aufl. 1981, Bd. II Seite 435). Sporenabtötende Präparate wie Perameisensäure und Ethylenoxid sind als besonders toxisch, z. T. als cancerogen einzuordnen. Phenole besitzen deutliche Lücken im Wirkungsspektrum und sind in der Regel unangenehm geruchsintensiv. Hypochlorite werden ungeachtet der erheblichen Anwenderunfreundlichkeit eingesetzt. Die Wirkung von Alkoholen gegen Hepadnaviren ist nicht sicher beweisbar, deshalb werden sie in Risikobereichen mit anderen Wirkstoffen eingesetzt. Auch bei den bisher verwendeten kationischen Tensiden und den Carbobetainen bestehen Wirkungslücken.

Wirkungen gegen ausgewählte Viren wurden nachgewiesen für:

- Aldehyde (Formaldehyd, Glutaraldehyd);
- Alkohole (Ethanol, Propanol);
- Halogenverbindungen (besonders Hypochlorite);

- Peressigsäure.

Bei der Bewertung der Mittel zur Keimabtötung stellen Viren und Bacteriensporen ein Hauptkriterium dar.

Als Testviren sind sehr häufig eingesetzt worden:

Influenzaviren, Vacciniaviren, Poliomyelitisviren;

dann folgen: Coxsackieviren, ECHO-Viren, Adenoviren, Herpesviren. Influenza- und Vacciniaviren sind in ihrem Verhalten gegenüber Desinfektionsmitteln als etwa gleich zu betrachten - trotz z. B. unterschiedlicher Ätherresistenz. Als besonders resistent gelten Hepadnaviren.

Ziel der Erfindung

Ziel der Erfindung sind solche Maßnahmen, die das antimikrobielle Spektrum erweitern, eine multivalente Keimabtötung auf chemischem bzw. physikochemischem Wege bei Beachtung anwendungstechnischer Kriterien erlauben, und zwar gleichzeitig gerichtet gegen Bacterien, Pilze und Viren sowie gegen andere Keime, wobei unter Anpassung an unterschiedliche Anwendungsbedingungen eine sichere Keimabtötung erreicht und einer toxischen Schädigung beim Anwender entgegengewirkt wird. Die Herstellung sowohl der Wirkstoffe als auch der Anwendungsformulierungen soll einfacher und kostengünstiger sein als bei den bekannten Wirkstoffen und Formulierungen.

Darlegung des Wesens der Erfindung

Die Aufgabe, die durch die Erfindung gelöst wird, besteht darin, solche Mittel und Maßnahmen anzugeben, die eine multivalente Keimabtötung/Inaktivierung von Keimen ohne Reaktivierung der Keime erreicht. Diese Maßnahmen sind ausgerichtet auf

- hohe Wirksamkeit (gegen Viren, Bacterien, Sporen, Wurmeier);
- Anwendungsfreundlichkeit (wenig bzw. nicht hautreizend, keine Korrosionswirkung, gewisse Reinigungswirkung, geringe Geruchsbelästigung, Wasserlöslichkeit);
- günstige Eigenschaften (toxikologische, cancerogene, mutagene, teratogene und ökologische Kriterien);
- Berücksichtigung des Kapillarproblems bzw. des Eindringens in Poren bzw. rauhe Flächen;
- Schmutzauflösung, Auflösung von Blutbeimengungen;
- ökonomische und rohstoffseitige Produzierbarkeit.

Der Zerfall oder Abbau der Wirkstoffe soll über ebenfalls microbicide Zwischenstufen oder Spaltprodukte verlaufen, um so eine möglichst langanhaltende und breite Wirkung zu erzielen.

Die Aufgabe wird erfindungsgemäß dadurch gelöst, daß die Keimminderung/Desinfektion durch Hydroxymethyl-sulfonylmethyl-pyrrolidinium-sulfobetain der Formel I oder durch die Bishydroxyalkyl-sulfonylmethylpyrrolidinium-sulfobetaine der Formeln II und III in wäßrigen Lösungen in Gesamtkonzentrationen zwischen 0,01 bis 2 Gewichtsprozent, welche zur synergistischen Wirkungssteigerung Methanol, Ethanol, ein Propanol, ein Hexanol oder 2-Ethylhexanol und/oder Emulgatoren aus der Reihe der anionischen Tenside enthalten können, vorgenommen wird.

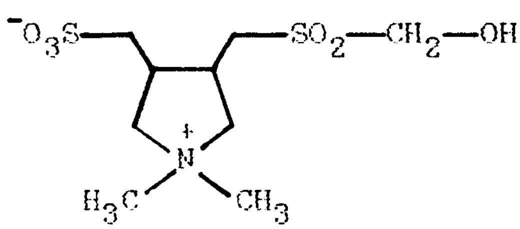

(I)

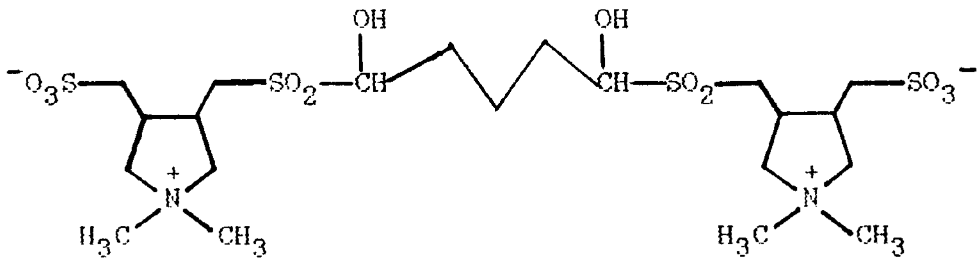

(II)

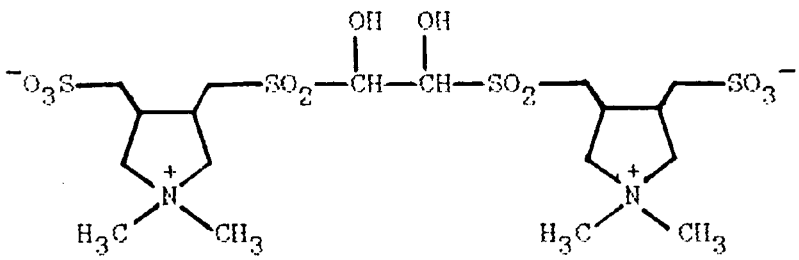

(III)

Die erfindungsgemäßen Verbindungen I bis III können in besonders ökonomischer und einfacher Weise durch Einwirkung der Aldehyde Formaldehyd, Glutardialdehyd und Glyoxal auf das ebenfalls sehr leicht in Wasser nach DD-PS 225 128 synthetisierbare 1,1-Dimethyl-3-sulfinsäuremethyl-4-sulfomethyl-pyrrolidiniumbetain gewonnen werden.

Im Konzentrationsbereich von 0,1 bis 2 Gewichtsprozent Wirkstoff wird eine Auflösung von Blutverschmutzungen erreicht.

Überraschenderweise wurde gefunden, daß hochresistente Keime (z. B. Hepatitis-B-Viren) durch die Kombination des polyfunktionellen Sulfobetains der Formel I mit Alkoholen (Methanol, Ethanol, Propanole) inaktiviert werden; ein solcher synergistischer wirkungssteigender Effekt tritt erfindungsgemäß in Lösungen ein, welche 20 bis 99 Vol.-% Wasser und 1 bis 80 Vol.-% Alkohol enthalten. Gegen Hepatitis-B-Viren werden 15 bis 80 Vol.-% Methanol, Ethanol oder Propanol in Kombination mit den erfindungsgemäßen Sulfobetainen eingesetzt. Durch diese günstige Kombinierbarkeit ist es möglich, das Verfahren zur multivalenten Keimabtötung unterschiedlichsten Anwendungsbedingungen anzupassen. Dadurch ist es auch möglich, spezifische Wirkungen gegen Bacterien, Sporen, Pilze, Viren, parasitische Protozoen und Wurmeier zu erzielen.

Eine synergistische Wirkungssteigerung wird ebenfalls dadurch erreicht, daß die erfindungsgemäßen Wirkstoffe in Wasser in Konzentrationen von 0,01 bis 2 Gewichtsprozent unter Zusatz von Emulgatoren aus der Reihe der anionischen Tenside, wie zum Beispiel Natrium-alkan-sulfonate mit Kettenlängen von $C_{10}$ bis $C_{18}$ oder Alkyl-benzol-sulfonate in Konzentrationen von 0,01 bis 2 Gewichtsprozent eingesetzt werden. Durch Zusatz der genannten Emulgatoren zu wäßrig/alkoholischen Wirkstofflösungen in den angegebenen Konzentrationen wird eine Steigerung des synergistischen Effekts erreicht.

Die erfindungsgemäßen Lösungen eignen sich zur Hautdesinfektion ebenso wie zur Keimminderung auf harten oder rauhen Oberflächen, beispielweise Geräten, lackierten Flächen, Metallen, Emaille und Keramik.

Die erfindungsgemäßen Desinfektionsmittellösungen wirken im Neutralbereich in der Anwendungskonzentration nicht hautreizend, nicht korrosiv, zeigen keinen unangenehmen Geruch, sind nicht toxisch und sowohl als Substanz als auch in Lösung problemlos unbegrenzt lagerfähig. Mit anderen wäßrigen oder wäßrigalkoholischen Mitteln sind sie im Bedarfsfalle kombinierbar. Durch Alkalien werden die Wirkstoffe zersetzt, ergeben hierbei jedoch microbicide Spaltprodukte, die ebenfalls noch zur Keimminderung beitragen. Durch vorgenanntes Eigenschaftsbild sind sie herkömmlichen Desinfektionslösungen in Wirkung und Gebrauchswert überlegen und können kostengünstig hergestellt werden.

Ausführungsbeispiele

Beispiel 1

Zu einem Microorganismengemisch mit $10^9$ Keimen pro ml in stark viskosem Nährmedium aus Bacillus subtilis, Endomycopsis bispora einschließlich deren Sporen, wird 1,1 Dimethyl-3-hydroxymethyl-sulfonylmethyl-4-sulfomethyl-pyrrolidinium-betain der Formel I in einer Endkonzentration von 0,2 Gewichtsprozent zugemischt und 30 min inkubiert. Nach der Entaktivierung des Wirkstoffes durch Verdünnen mit sterilem Leitungswasser erfolgt die Prüfung auf Sterilität mit den Nährböden gemäß AB-DDR, Bd. I, X, 4.0.1.ff und 7.0.1.ff.

Ergebnisse

mit Wirkstoff I : steril
Kontrolle ohne Wirkstoff : Keimwachstum
Versuchswiederholung mit den Verbindungen II und III ergab das gleiche Ergebnis.

Beispiel 2

Zu Hühnerblut, das 10 Vol.-% Citratpuffer enthält, werden Influenzaviren Typ A Stamm A/PR/8/34 in Allantoisflüssigkeit gegeben. Nach gründlichem Vermischen wird dazu ein solches Volumen einer 10 %igen Lösung des Hydroxymethylsulfons der Formel I in 10 %igem Ethanol gegeben, daß die Endkonzentration an Wirkstoff 0,25 Gewichtsprozent beträgt. Nach erneutem Durchmischen und einer Einwirkungszeit von 10 min wird durch Zugabe des mindestens 100-fachen Volumens einer gepufferten NaCl-Lösung entaktiviert.
Damit werden nach den üblichen Verfahren für die Vermehrung von influenzaviren vorbebrütete Hühnereier beimpft. Von diesen Eiern wird nach einer Inkubationszeit von mindestens 48 Stunden die Allantoisflüssigkeit steril entnommen und die Virusvermehrung im Vergleich zur mit NaCl-Lösung beimpften Kontrolle und zu den Bruteiern ohne Wirkstoff mit Hilfe des Hämagglutinationstestes vergleichend beurteilt.

Ergebnisse

mit Wirkstoff I : keine Virusvermehrung
ohne Wirkstoff : volle Virusvermehrung
Die Versuchswiederholung mit Wirkstoff II ergab ein gleichgutes Resultat.

Beispiel 3

Zu einer gepufferten Virussuspension, die mindestens $10^4$ infektiöse Einheiten pro ml Adenovirus (human) Typ 2 enthält, wird ein solches Volumen einer 10 %igen Lösung von Wirkstoff I in 25 %igem Propanol gegeben, daß die Endkonzentration an Wirkstoff 0,4 % beträgt.

Nach dem Durchmischen und einer Einwirkungszeit von 30 min wird durch Zugabe des mindestens 400-fachen Volumens einer lactalbuminhaltigen Phosphatpufferlösung entaktiviert.

Damit wird nach den üblichen Verfahren eine für die Vermehrung dieser Adenoviren geeignete permanente Zellinie beimpft. Von jedem Kulturgefäß werden mehrere Subkulturen angelegt und die Virusreplikation im Vergleich zur unbeimpften Kontrolle und zu den Zellkulturen mit Adenovirus ohne Wirkstoff mit Hilfe des cytopathischen Effektes (CPE) sowie der Immunfluoreszenz vergleichend beurteilt.

Ergebnisse

mit Wirkstoff I : kein CPE
ohne Wirkstoff : CPE
Vergleichsversuche mit den Wirkstoffen II und III verliefen analog.

Beispiel 4

Inaktivierung von Hepatitis-B-Oberflächenantigen (HBsAg) im Suspensionstest:

Gleiche Teile eines HBsAg-haltigen Serumpools und einer 0,1 %igen Lösung der Substanz I in 70 %igem Ethanol werden gemischt. Nach einer Einwirkungszeit von 30 min werden die Proben durch eine Verdünnung von 1 : 100 inaktiviert. Die Bestimmung der Restaktivität des HBsAg erfolgt mit einem Radioimmunoassay im Vergleich zu einem Kontrollansatz.

| | | Restaktivität in % (Kontrolle = 100 %) |
|---|---|---|
| 0,1 % | Wirkstoff I | |
| | in 70 %igem Ethanol | 4,5 % |
| | in Wasser | 79,0 % |

Beispiel 5

Mäuseschwänzchentest

10 mm lange Schwanzstücke werden nach gründlicher Vorreinigung 5 min in einen HBsAg-haltigen Serumpool eingelegt und 30 min bei Zimmertemperatur angetrocknet. Zum Desinfektionsversuch werden die Schwanzstückchen einzeln in 50 ml Meßbecher, deren Boden mit Glasperlen bedeckt ist, eingelegt, 1 ml Desinfektionslösung (0,1 %ige bzw. 0,05 %ige Lösung von Wirkstoff I in 50 %igem n-Propanol bzw. 0,1 %ige Lösung von Wirkstoff I in 0,1 %igem Emulgator E 30) zugegeben und 2 bzw. 30 min apparativ geschüttelt. Danach wird die Probe 1:50 verdünnt und die Restaktivität des HBsAg mit einem Radioimmunoassay bestimmt. Die Schwanzstücke werden mit 3 ml physiologischer Kochsalzlösung übergossen und erneut 5 min geschüttelt. Die Restaktivität in der Spülflüssigkeit wird wiederum mit einem Radioimmunoassay bestimmt.

Ergebnisse in % der Kontrolle

D = Desinfektionslösung
S = Spüllösung

Restaktivität des HBsAg:

| | Einwirkungszeit | | | |
|---|---|---|---|---|
| | 2 min | | 30 min | |
| | D | S | D | S |
| 0,1 % I in 50 %igem Propanol | 3 | 15 | 2 | 23 |
| 0,05 % I in 50 %igem Propanol | 13 | 22 | 23 | 10 |
| 0,1 % I in 0,1 %igem Emulgator E30 (Alkansulfonatgemisch) | 3 | 36 | 2 | 27 |

Beispiel 6

Meerschweinchenhauttest

Aus rasierter Rückenhaut von Meerschweinchen ausgestanzte Fellstücke werden um Knöpfe mit einer planen Oberfläche gelegt (Durchmesser 2,5 cm), auf der Rückseite mit chirurgischem Nahtmaterial vernäht und durch in die Knopflöcher eingeführte Holzstäbchen fixiert. Für einen Test werden jeweils 2 derart präparierte Keimträger benötigt.
Ein Keimträger wird mit 0,05 ml HBsAg-haltigem Serum beschickt und 30 min bei Raumtemperatur angetrocknet. Danach werden 0,5 ml der zu prüfenden Desinfektionslösung hinzugefügt und mit Hilfe des zweiten Keimträgers waschende Bewegungen ausgeführt. Nach einer Einwirkungszeit von 2 bzw. 30 min wird die Desinfektionslösung abgewaschen und anschließend werden die Keimträger 5 min mit 3 ml 0,9 %iger Kochsalzlösung gespült. Die Restaktivität des HBsAg wird mit einem Radioimmunoassay bestimmt.

Ergebnisse

0,1 %ige Lösung von Wirkstoff I in 50 %igem n-Propanol Restaktivität des HBsAg in % der Kontrolle:

| Einwirkungszeit 2 min | Einwirkungszeit 30 min |
|---|---|
| 1,9 % | 3,9 % |

Beispiel 7

Herstellung des Wirkstoffes I

1,1-Dimethyl-3-hydroxymethyl-sulfonylmethyl-4-sulfomethyl-pyrrolidiniumbetain der Formel I als konzentrierte wäßrige Lösung

Beispiel für einen 200-Liter-Ansatz (169 mol des Wirkstoffes I)

Man bereite folgende Lösungen:

1. 54,56 kg 50 %ige Lösung von Dimethyl-diallyl-ammonium-chlorid (169 mol, hergestellt nach DD-PS 136 497, Beispiel 2)
2. 99,3 kg 39 %ige $NaHSO_3$-Lösung (373,7 mol)
3. 2,35 kg $Na_2S_2O_8$ in 4,74 kg Wasser
4. 5,4 kg 37 %ige Salzsäure
5. 11,25 kg 37 %ige Salzsäure
6. 20,95 kg 27,5 %ige Formalinlösung (192 mol)
7. 7,58 kg Wasser

Man versetzt die Lösung 1 mit den Lösungen 2 und 4 und gibt danach unter heftigem Rühren innerhalb von 15 bis 20 min die Lösung 3 in dünnem Strahl zu. Hierbei erwärmt sich das Reaktionsgemisch auf ca 70 °C. Die Lösung enthält nun 1,1-Dimethyl-3-sulfinsäuremethyl-4-sulfomethyl-pyrrolidiniumbetain als Natriumsalz, aus dem man nach dem Abkühlen auf 20 °C durch Zugabe der Lösung 5 die Sulfinsäure freisetzt. Diese setzt man durch Zugabe der Lösung 6 und 7 zum Wirkstoff I um. Man kann hierzu 1 bis 2 Stunden auf 60 °C erwärmen oder die Umsetzung durch Stehenlassen bei Zimmertemperatur in 48 Stunden zu Ende führen. Man erhält 206 kg 25%ige Lösung von I, welche sauer reagiert, NaCl enthält und für weitere Umsetzungen oder Applikationen direkt einsetzbar ist. Zur Verwendung als Microbicid erfolgt pH-Einstellung der Lösungen auf pH-Werte unter 7 mittels Puffersubstanzen.

Beispiel 8

Herstellung des Wirkstoffes II

0,5 mol (169,37 g) 1,1-Dimethyl-3-sulfinsäuremethyl-4-sulfomethyl-pyrrolidinium-betain (80 %iges Produkt, hergestellt nach DD-PS 225 128)

0,25 mol Glutardialdehydlösung (45 %ig = 55,57 g) und 340 g Wasser werden auf 70 °C erwärmt und nach völliger Auflösung 24 Stunden stehengelassen. Man engt im Vakuum ein und fällt aus der aufkonzentrierten Lösung den Wirkstoff II durch Zugabe von 130 g Ethanol aus.

Er kristallisiert als Dihydrat mit 2 mol Kristallwasser. Ausbeute 169 g, d. h. quantitativer Umsatz, berechnet auf 0,25 mol des Dihydrates (MG 678,8).

Beispiel 9

Herstellung des Wirkstoffes III

0,3 mol (92,1 g) 1,1-Dimethyl-3-sulfinsäuremethyl-4-sulfomethyl-pyrrolidiniumbetain (80 %iges Produkt nach DD-PS 225 128) und 0,15 mol Glyoxallösung (21,76 g 40 %ige wäßrige Lösung) werden zusammen mit 75 g Wasser kurze Zeit zum Sieden erwärmt. Die erhaltene, fast klare Lösung wird warm filtriert und das Produkt durch Abkühlen (mehrere Tage) kristallisiert. Nach kurzem Waschen mit Wasser wird getrocknet. Ausbeute 80,8 g (90 % der Theorie).

Beispiel 10

Eine gepufferte Suspension von Adenoviren (human) Typ 2, die einen hohen Anteil an Ballaststoff in Form von 25 % Albumin (human) hat, wird mit 0,30 % Wirkstoff I, 0,25 % Emulgator E30 und 20 % Propanol (alle Substanzen bezogen auf die Endkonzentration) versetzt. Das weitere Vorgehen erfolgt gemäß Beispiel 3.

Ergebnisse

mit Wirkstoff I : kein CPE
ohne Wirkstoff : CPE

Vergleichsversuche mit den Wirkstoffen II und III verliefen analog.

## Patentansprüche

1. Desinfektionsmittellösung zur verbesserten Keimabtötung, gekennzeichnet dadurch, daß die Keimminderung, keimmindernde Reinigung und/oder Desinfektion durch 1,1-Dimethyl-3-hydroxymethylsulfonylmethyl-4-sulfomethyl-pyrrolidiniumbetain der Formel I oder durch die Bis-3-hydroxyalkylsulfonylmethyl-4-sulfomethyl-pyrrolidiniumbetaine der Formeln II und III

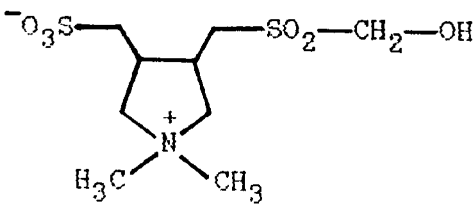

(I)

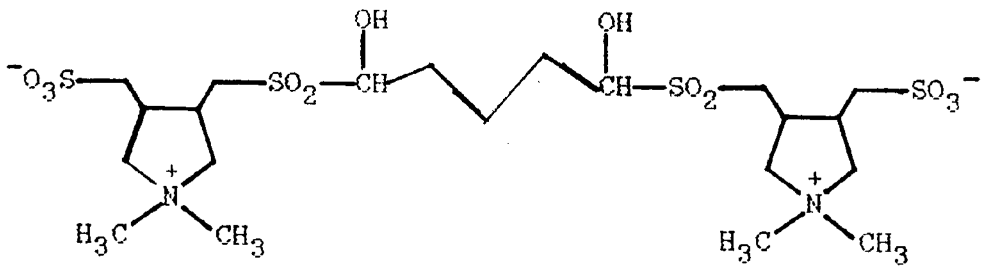

(II)

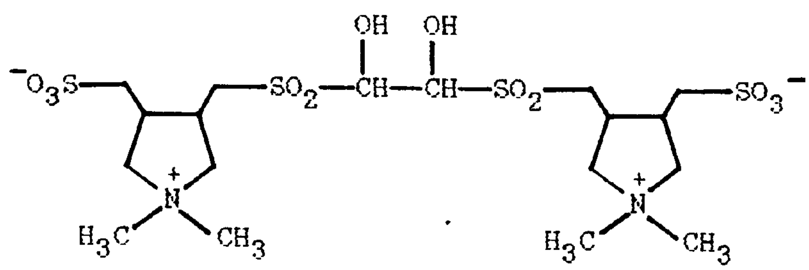

(III)

erfolgt, welche in Wasser in Konzentrationen von 0,01 bis 2 Gewichtsprozent angewandt oder zur synergistischen Wirkungssteigerung in gleicher Konzentration in Wasser/Alkoholmischungen mit einem Anteil von 20 bis 99 Vol.-% Wasser und 1 bis 80 Vol.-% Alkohol gelöst werden, wobei der wäßrigen oder wäßrig/alkoholischen Wirkstofflösung zur synergistischen Wirkungssteigerung bzw. Erhöhung des synergistischen Effekts Emulgatoren aus der Reihe der anionischen Tenside in Konzentrationen von 0,01 bis 2 Gewichtsprozent zugesetzt werden können.

2. Desinfektionsmittellösung nach Anspruch 1, dadurch gekennzeichnet, daß als Alkohol Methanol, Ethanol, ein Propanol, ein Hexanol oder 2-Ethylhexanol verwendet wird.

3. Desinfektionsmittellösung nach Anspruch 1 und 2, dadurch gekennzeichnet, daß zur Hautdesinfektion mit spezifischer Wirksamkeit gegen Hepatitis-B-Viren die Wirkstoffe in Wasser/Alkohol-Mischungen mit einem Alkoholgehalt von 15 bis 80 Volumenprozent gelöst werden.

4. Desinfektionsmittellösung nach Anspruch 1, dadurch gekennzeichnet, daß zum Erreichen einer nahezu vollständigen Auflösung von Blutverschmutzungen eine Wirkstoffkonzentration von 0,1 bis 2 Gewichtsprozent angewandt wird.

5. Desinfektionsmittellösung nach Anspruch 1, dadurch gekennzeichnet, daß als Emulgatoren zur synergistischen Wirkungssteigerung Natrium-alkan-sulfonate mit Kettenlängen von $C_{10}$ bis $C_{18}$ oder Alkyl-benzolsulfonate im Konzentrationsbereich von 0,01 bis 2 Gewichtsprozent der wäßrigen oder wässrig/alkoholischen Wirkstofflösung zugesetzt werden.

## Claims

1. A disinfectant solution with improved germicidal action, wherein the germ diminishing action, the germ diminishing cleaning and/or disinfection is achieved by reacting 1,1-dimethyl-3-hydroxymethylsulfonylmethyl-4-sulfomethyl-pyrrolidiniumbetaine of formula I or bis-3-hydroxyalkylsulfonylmethyl-4-sulfomethyl-pyrrolidinium-betaines of formulas II and III

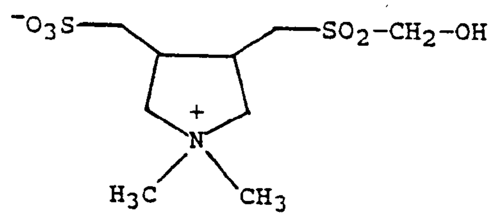

(I)

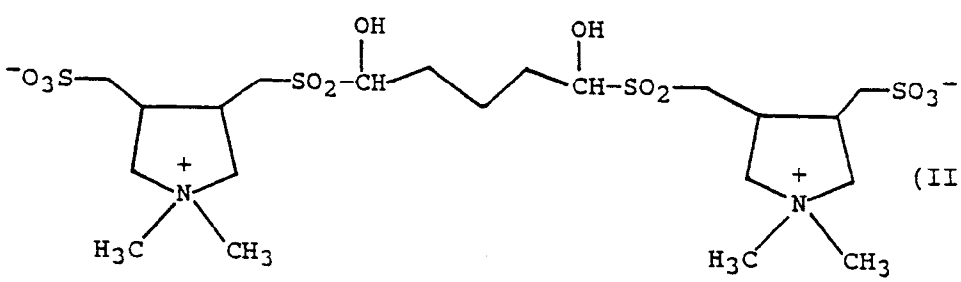

(II)

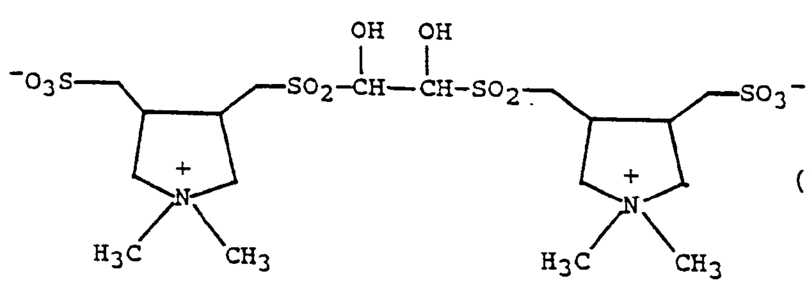

(III)

with water in concentrations of 0,01 to 2 percent by weight or by dissolving, for synergistic fortification, in equal concentrations in water/alcohol mixtures with a portion of 20 to 99 percent by volume of water

and 1 to 80 percent by volume of alcohol, and wherein for synergistic fortification or enhancing their synergistic action emulsifying agents from the family of anionic surfactants in concentrations of 0,01 to 2 percent by weight may be added to the aqueous or aqueous/alcoholic solution of the said agent.

**2.** A disinfectant solution according to claim 1, wherein the alcohol is methanol, ethanol, propanol, hexanol or 2-ethylhexanol.

**3.** A disinfectant solution according to claim 1 and 2, wherein for skin disinfection with particular activity against hepatitis B viruses, the said agents are dissolved in aqueous/alcoholic mixtures with a portion of 15 to 80 percent by volume of alcohol.

**4.** A disinfectant solution according to claim 1,wherein for achieving a nearly complete dissolution of blood stains a concentration of the said agent of 0,1 to 2 percent by weight is applied.

**5.** A disinfectant solution according to claim 1, wherein for synergistic fortification as emulsifying agents sodium-alkane-sulfonates with chain lengths of $C_{10}$ to $C_{18}$ or alkyl-benzene-sulfonates in concentrations of 0,01 to 2 percent by weight are added to the aqueous or aqueous/alcoholic solution of the said agent.

## Revendications

**1.** Solution désinfectante à action germicide améliorée, caractérisée par le fait que la réduction des germes, une désinfection et/ou un nettoyage à action réductrice des germes sont obtenus à l'aide de diméthyl 1,1-hydroxyméthylsulfonylméthyl-3-sulfométhyl-4-pyrrolidinium bétaïne de formule I ou de bis [hydroxyalkylsulfonylméthyl-3, sulfométhyl 4- pyrrolidinium bétaïne) de formules II et III,

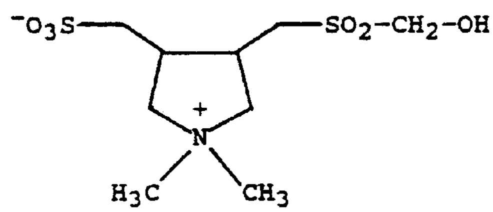

(I)

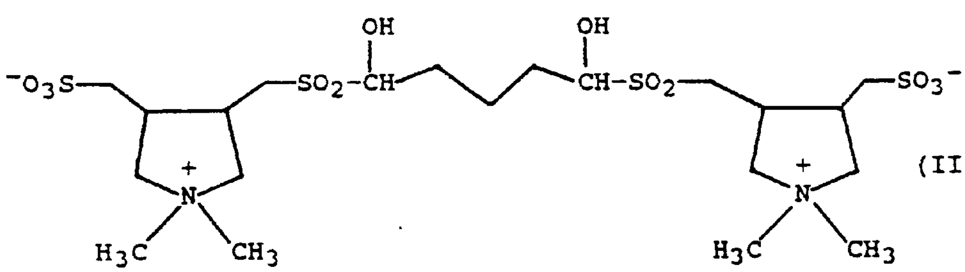

(II)

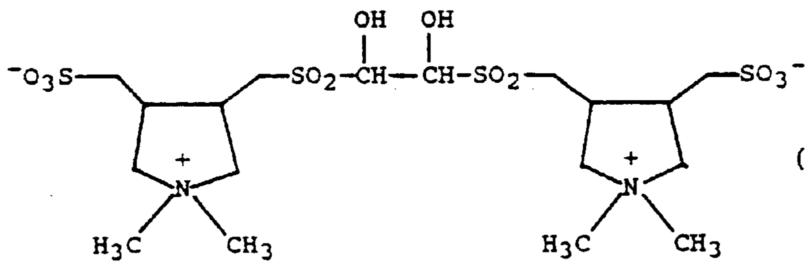

(III)

utilisées dans l'eau à concentrations de 0,01 à 2% en poids, ou pour une augmentation synergétique de l'action en solutions à concentrations égales dans des mélanges eau/alcool de 20 à 99% en volume d'eau et de 1 à 80% en volume d'alcool, la solution de substance active en phase aqueuse ou aqueuse/alcoolique pouvant être additionnée, pour un renforcement ou une augmentation synergétique des effets synergétiques, d'émulsifiants de la série des agents tensio-actifs anioniques à raison de 0,01 à 2% en poids.

2. Solution désinfectante selon la revendication 1 caractérisée par le fait qu'elle utilise, comme alcool, du méthanol, de l'éthanol, un propanol, un hexanol ou de l'éthyl 2-hexanol.

3. Solution désinfectante selon la revendication 1 et 2 caractérisée par le fait que, pour la désinfection de la peau avec une efficacité spécifique contre le virus de l'hépatite-B, on dissout la substance active dans des mélanges eau/alcool avec une teneur en alcool de 15 à 80% en volume.

4. Solution désinfectante selon la revendication 1 caractérisée par le fait que, pour parvenir à une élimination pratiquement totale des souillures sanguines, on utilise une concentration en substance active de 0,1 à 2% en poids.

5. Solution désinfectante selon la revendication 1 caractérisée par le fait que, comme émulsifiants pour le renforcement synergétique de l'action, on ajoute à la solution aqueuse ou aqueuse/alcoolique un sulfonate d'alcane-sodium avec des longueurs de chaînes de $C_{10}$ à $C_{18}$ ou un sulfonate d'alkyl-benzol dans des gammes de concentration de 0,01 à 2% en poids de solution.